# EUROPEAN PATENT SPECIFICATION

(11) **EP 2 991 554 B1**
(45) Date of publication and mention of the grant of the patent: **22.04.2020**
(21) Application number: 14725244.9
(22) Date of filing: 11.04.2014
(51) Int. Cl.: A61B 6/04, A61B 6/00

(54) **IMAGING SYSTEM SUBJECT SUPPORT TABLETOP DEFLECTION DELTA CORRECTION**
ABLENKUNGSDELTA-KORREKTUR FÜR EINE PATIENTENTRÄGERTISCHPLATTE IN EINEM BILDGEBUNGSSYSTEM
CORRECTION DE DELTA DE DÉFORMATION DE PLATEAU DE TABLE DE SUPPORT DE SUJET DE SYSTÈME D'IMAGERIE

(30) Priority: 01.05.2013 US 201361817888 P
(43) Date of publication of application: 09.03.2016
(73) Proprietor: Koninklijke Philips N.V., 5656 AG Eindhoven (NL)
(72) Inventor: DONG, Shufang, 5656 AE Eindhoven (NL)
(74) Representative: Philips Intellectual Property & Standards
(86) International application number: PCT/IB2014/060645
(87) International publication number: WO 2014/177958

(56) References cited:
- DE-A1-102007 060 690
- US-A1- 2002 122 575
- US-A1- 2010 287 703

## Description

The following generally relates to an imaging system subject support that supports a subject or object in an examination region of the imaging system in a cantilevered position in which a weight of a tabletop of the support and the subject or object thereon causes the cantilevered end of the subject support to deflect as a function of at least the extent of the cantilever.

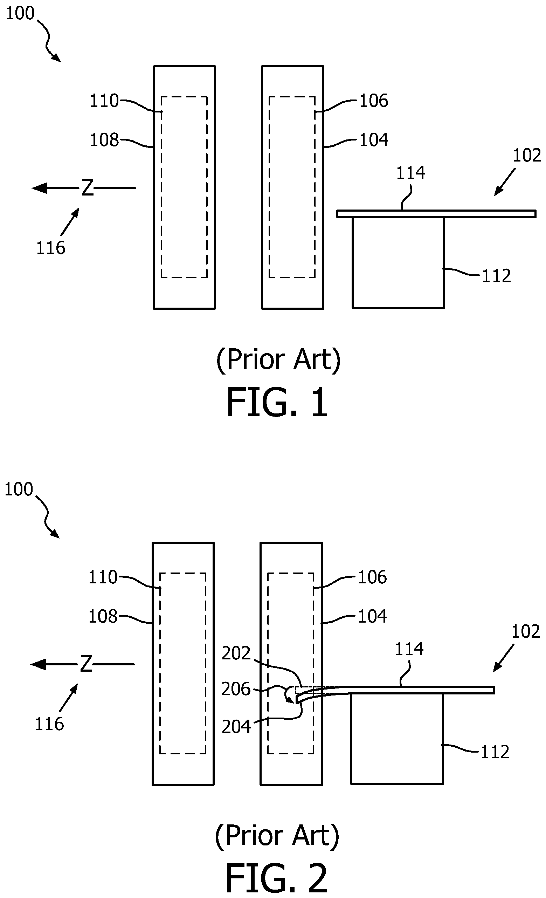

FIGURE 1 shows an example dual scanner imaging system 100. The imaging system 100 includes a subject support 102, a first gantry portion 104 (with a first examination region 106) and a second gantry portion 108 (defining a second examination region 110). The subject support 102 includes a base 112 configured to translate vertically and a tabletop 114, moveably attached to the base 112, that is configured to translate back and forth horizontally along a z-axis 116 direction. The tabletop 114 loads and unloads a subject or object in the examination regions 106 and 110. The tabletop 114, when cantilevered, deflects under the weight of at least the tabletop 114.

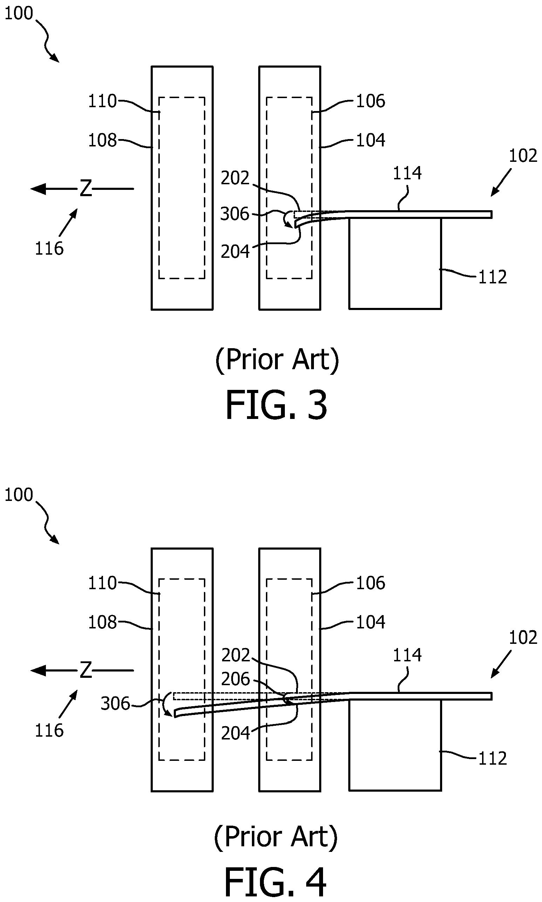

This is shown in FIGURES 2 and 3, which show the cantilevered tabletop 114 in a non-deflected state 202 and a deflected state 204 in a same gantry portion 104 at two different locations in the examination region 106. As shown, the amount of the deflection depends on a length of the cantilevered portion. As such, a deflection 206 of FIGURE 2 is less than a deflection 306 of FIGURE 3. The difference between the deflections 206 and 306, or a deflection delta, represents a vertical height difference of the tabletop 114 at the two different locations. FIGURE 4 shows this in connection with a same corresponding location in the two gantry portions 104 and 108.

As a consequence, a structure on the tabletop 114 will be at a different height from scan to scan during axial (step and shoot) scanning with one of the gantry portions 104 or 108, which may introduce step artifact into the reconstructed images. Furthermore, with helical scanning with one of the gantry portions 104 or 108, the deflection delta may degrade image spatial resolution. Moreover, scanning with both of the gantry portions 104 and 108, the deflection delta may result in miss-registration between the images of each of the gantry portions 104 and 108.

One approach to reducing the deflection delta is to stiffen the tabletop 114. Unfortunately, this increases costs and/or tabletop thickness, which changes the attenuation profile of the tabletop and can degrade image quality and/or dose efficiency. Another approach has been to use an auxiliary "catcher" that engages and carries the end of the cantilevered tabletop to prevent the tabletop further deflection. However, the catcher is an additional device, which adds costs and needs to be synchronized with the base vertical motion. The catcher also consumes additional room space.

In yet another approach, optics (e.g., a laser or an optical fiber) are used to estimate deflection. With this approach, a transmitter transmits an optical signal along a path (horizontal and/or vertical to the tabletop) that changes based on tabletop deflection and receives a return optical signal indicative of a change in the path. A deflection signal is determined based thereon and is provided to the reconstructor along with the acquired data. Unfortunately, this approach requires incorporation of at least one optical transmitter and receiver into the imaging system, which may increase overall system cost and complexity.

Aspects described herein address the above-referenced problems and others.

Described herein is an approach to estimate a weight of a subject or object begin scanned and use the estimated weight to predict a deflection delta, which can be used to adjust a vertical height of the tabletop and/or correct for the deflection delta during reconstruction, and/or correct for the deflection delta when registering images.

Provision of a deflection delta predictor that predicts a deflection delta of the tabletop between at least two different positions in the examination region based on an estimation of the patient's weight is known e.g. from US 2002/122575, DE 10 2007 060690 and US 2010/287703.

In one aspect, an imaging system includes a first scanner with a first examination region and a subject support. The subject support includes a tabletop that positions a subject or object at at least two different positions in the examination region. A deflection of the tabletop is different at the at least two different positions. The subject support further includes a base that positions the tabletop vertically in the examination region. A deflection delta predictor predicts a deflection delta of the tabletop between the at least two different positions. The predicted deflection delta is used to correct for the deflection delta.

In another aspect, a method includes estimating a weight of a subject or object on a tabletop of a subject support of an imaging system. The method further includes obtaining a vertical position of a base of the subject support. The method further includes obtaining a horizontal position of the tabletop in the examination region. The method further includes predicting a deflection delta of the tabletop based on the weight, the vertical position, and the horizontal position. The method further includes correcting for a deflection delta for the tabletop based on the predicted deflection delta.

In another aspect, a computer readable storage medium includes one or more computer executable instructions, which, when executed by a processor of a computing system, causes the processor to: correct for a deflection delta of a tabletop of a subject support of an imaging system based on a weight of a subject or object on the tabletop, a vertical position of a base of the subject support, and a horizontal position of the tabletop.

The invention may take form in various components and arrangements of components, and in various steps and arrangements of steps. The drawings are only for purposes of illustrating the preferred embodiments and are not to be construed as limiting the invention.
FIGURE 1 schematically illustrates a prior art dual scanner imaging system including a subject support with a tabletop and base.
FIGURE 2 schematically illustrates a first deflection of the tabletop at a first location in one of the scanners of FIGURE 1.
FIGURE 3 schematically illustrates a second deflection, which is different from the first deflection, of the table at a second location in the one of the scanners of FIGURE 1.
FIGURE 4 schematically illustrates a first deflection of the tabletop at a location in one of the scanners and a second deflection, which is different from the first deflection, of the table at a corresponding location in the other of the scanners of FIGURE 1.
FIGURE 5 schematically illustrates an example imaging system, with one or more scanners, configured to correct for a deflection delta.
FIGURE 6 schematically illustrates an example in which weight is determined based on load cells affixed to the tabletop.
FIGURE 7 schematically illustrates an example in which weight is determined based on a load cell affixed to a screw nut of the horizontal drive system.
FIGURE 8 schematically illustrates an example in which weight is determined based on an electrical characteristic of the vertical drive system.
FIGURES 9 and 10 illustrate an example method for correcting for a deflection delta by adjusting a vertical position of a tabletop in one of a plurality of scanners of a multi-scanner imaging system.
FIGURES 11 and 12 illustrate an example method for correcting for a deflection delta by adjusting a vertical position of a tabletop between two scanners of a multi-scanner imaging system.
FIGURES 13 and 14 illustrate an example method for correcting for a deflection delta by adjusting a vertical position of a tabletop in a single scanner imaging system.
FIGURE 15 illustrates an example method for correcting for a deflection delta by adjusting a vertical position of a tabletop during data acquisition.
FIGURE 16 illustrates an example method for correcting for a deflection delta during image reconstruction.
FIGURE 17 illustrates an example method for correcting for a deflection delta during image registration.
The following describes an approach in which a estimated subject or object weight of a subject or object being scanned is used to predict a subject support tabletop deflection delta (or a change in deflection of a same region of the tabletop for different tabletop horizontal positions), which is used to correct for the deflection delta by adjusting a vertical height of the subject support and/or correct for the deflection delta during reconstruction and/or image registration.

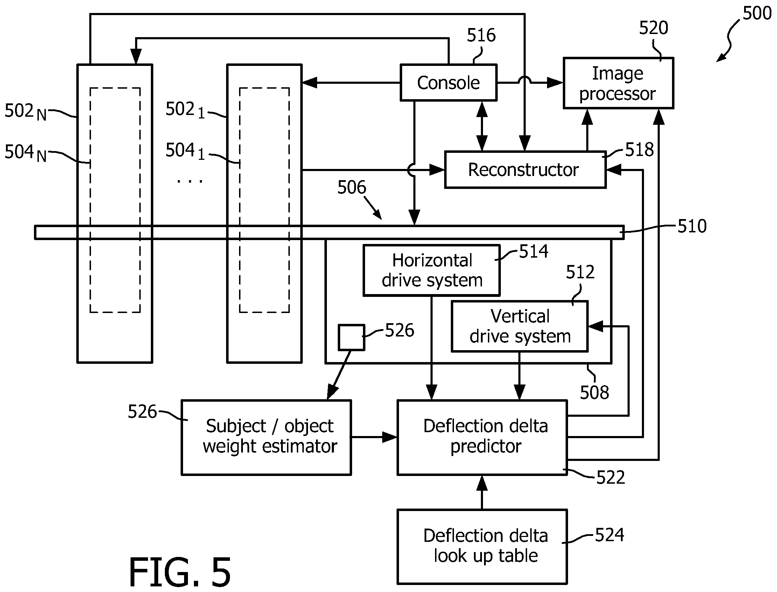

FIGURE 5 schematically illustrates an imaging system 500 with N scanners 502₁, ..., 502_{N} (collectively referred to as scanner(s) 502),where N is an integer equal to or greater than one. Where N=1, the scanner(s) 502 can include a computed tomography (CT), a positron emission tomography (PET), includes a magnetic resonance (MR), a single photon emission tomography (SPECT), and/or other scanner. Where N>1, the scanner(s) 502 can include two or more of the above scanners, including at least two different imaging modalities or at least two of the same imaging modalities.

The N scanners 502₁, ..., 502_{N} respectively include N examination regions 504₁, ..., 504_{N} (collectively referred to as examination region(s) 504). A subject support 506 includes a base 508 and a tabletop 510. The base 508 is vertically moveable and the tabletop 510 is horizontally moveable and configured to translate relative to the base 508, into and out of the examination region(s) 504. The tabletop 510, when cantilevered from the base 508, deflects under the weight of a subject and/or object (not shown) disposed thereon. The difference in deflection between any two different horizontal locations is a deflection delta.

A vertical drive system 512 (e.g., a controller, a motor, a drive apparatus, etc.) raises and lowers the base 508 and hence the tabletop 510 between predetermined upper and lower mechanical limits. A horizontal drive system 514 (e.g., a controller, a motor, a drive apparatus, etc.) translates the tabletop 510 between a fully retracted and a fully extended (or cantilevered) position. The upper and lower mechanical limits may vary depending on a current location of the tabletop 510, and the fully retracted and a fully extended positions may vary depending on a height of the base 508.

A computing system serves as an operator console 516, which includes human readable output devices such as a display and input devices such as a keyboard and/or mouse. Software resident on the console 516 allows the operator to control an operation of the imaging system 500. In the illustrated embodiment, this includes activating tabletop deflection delta correction, which includes determining a deflection delta and adjusting a vertical position of the tabletop 510 based thereon and/or employing the deflection delta during image reconstruction and/or image registration.

A deflection delta predictor 522 predicts the deflection delta. In the illustrated embodiment, this includes predicting the deflection delta based on a horizontal tabletop position, a base vertical position, and a weight of a subject or object on the tabletop 510, and a deflection delta (LUT) look up table 524. The deflection delta LUT 524 can be a predetermined four dimensional look up table calibrated with weight and vertical and horizontal position parameters determined during product development, manufacturing, installation, etc., which assigns a deflection delta value to the different input combinations.

A subject/object weight estimator 526 estimates the weight of the subject or object on the tabletop 510 based on certain information obtained by a device 526. The subject/object weight estimator 526 can be pre-calibrated with no load on the tabletop 510 and then estimates a weight of a subject or object on the tabletop 510 based on electrical information (e.g., a voltage, a current, etc.) from the vertical drive system 512. For example, the electrical information can be used to identify a weight in a pre-determined look up table.

In the illustrated embodiment, the predicted deflection delta can be used to correct for a deflection delta by adjusting a vertical height of the subject support 506 and/or correct for the deflection delta during reconstruction and/or image registration. For vertical height, as described in greater detail below the predicted deflection delta is provided to the vertical drive system 512, which can employ the predicted deflection delta to adjust or drive the height of the subject support 506. This can be done on the fly so that a same location of the tabletop 510 is at approximately a same height regardless of the horizontal position of the tabletop 510.

A reconstructor 518 reconstructs raw data from the N scanners 502₁, ..., 502_{N}, generating images of the scanned subject or object. Where deflection delta correction is activated for reconstruction, the reconstructor 518 employs the predicted deflection delta to correct for deflection delta. The predicted deflection delta can be included with the raw imaging detector data provided to the reconstructor 518 and/or otherwise provided to the reconstructor 518. The illustrated embodiment shows a single reconstructor 518 that reconstructs raw data from the N scanners 502₁, ..., 502_{N}. However, it is to be appreciated that each of the N scanners 502₁, ..., 502_{N} may have its own corresponding reconstructor.

An image processor 520 performs various image processing functions. For example, in one instance, the image processor 520 registers images generated by the reconstructor 518. This may include registering images generated by scanners 502 of different modalities. This may also include registering images generated by a same modality but different times, e.g., an initial and a follow up or subsequent scan. The image processor 520 can also registers images generated by another reconstructor, a simulator, an anatomical atlas, and/or other image. The image processor 520 can employ the predicted deflection delta to correct for vertical height differences between the images being registered.

The deflection delta predictor 522 and/or the subject/object weight estimator 526 can be implemented via a micro-processor(s) of a computer system(s), which executes a computer readable instruction(s). In one instance, the computer readable instruction(s) is encoded on computer readable storage medium such a physical memory and other non-transitory medium. Additionally or alternatively, at least one of the computer readable instructions can be carried by a carrier waver, a signal and other transitory medium.

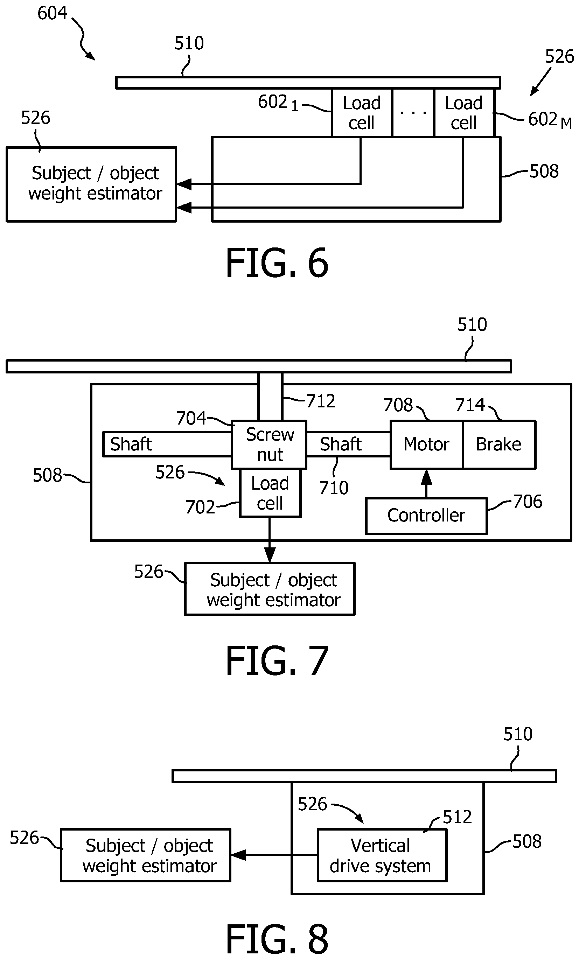

As briefly discussed above, the subject/object weight estimator 526 estimates a weight of the subject or object on the tabletop 510 based at least on an electrical characteristic of the vertical drive system obtained by the device 526. FIGURES 6 and 7 illustrate alternative weight estimation means of the device and FIGURE 8 discloses the subject/object weight estimator according to the invention.

In FIGURE 6, the device 526 includes at least two transducers (e.g., a load cell, etc.) that convert force into an electrical signal (e.g., a current, a voltage, a resistance, etc.). In this example, a first load cell 602₁ and an Mth load cell 602_{M} (where M = an integer equal to or greater than two) are located at a rear end 604 of the tabletop 501, which is the end opposing the end that enters the examination region(s) 504. The load cells 602₁ and 602_{M} are calibrated or "zeroed" based on a weight of the tabletop 510. When a subject or object is disposed on the tabletop 510, the output signal of the load cells 602 and 604 is indicative of weight of the subject or object. At least two load cells 602 and 604 account for load position difference on the tabletop 510. This approach and other load cell approaches provide a fast, stable and accurate reading.

In FIGURE 7, the device 526 includes a transducer (e.g., a load cell, etc.) that converts a force into an electrical signal (e.g., a current, a voltage, a resistance, etc.). In this example, the transducer includes a load cell 702 located in connection with a screw nut 704 of the horizontal drive system 514, which also includes, in this example, a controller 706, which controls a motor 708, which drives a shaft 710 to which the screw nut 704, which is affixed to the tabletop 510 via a coupling 712, translates along, and a motor brake 714. Likewise, the load cell 702 is calibrated or "zeroed" based on a weight of the tabletop 510, and when a subject or object is disposed on the tabletop 510, the output signal of the load cell 702 is indicative of weight of the subject or object.

In FIGURE 8, the device 526 includes the vertical drive system 512. For example, in one instance, the motor current of the motor of the vertical drive system 512 is used to estimate the weight. For this, the motor current can be the static vertical holding current. Then, by releasing the vertical holding brake and using the servo motor to hold the vertical position for a second or so, the motor current is sampled and averaged, and then compared with a calibrated value to extract the patient weight information. In alternative embodiments, the constant speed vertical motion current, or the horizontal motor current during acceleration and deceleration phase can be used to extract the weight. The motor current approach, generally, does not add any hardware and associated cost and/or require space.

In a variation, a combination of FIGURE 8 with FIGURES 6 and/or 7 can be used to estimate the weight. Additionally this information may be manually entered via the console 516. In case of transducer error, a noisy motor current, a noisy transducer signal, an out of the normal reading bounds, etc., the deflection data can be ignored, discarded, etc., and not used for deflection delta compensation and/or correction.

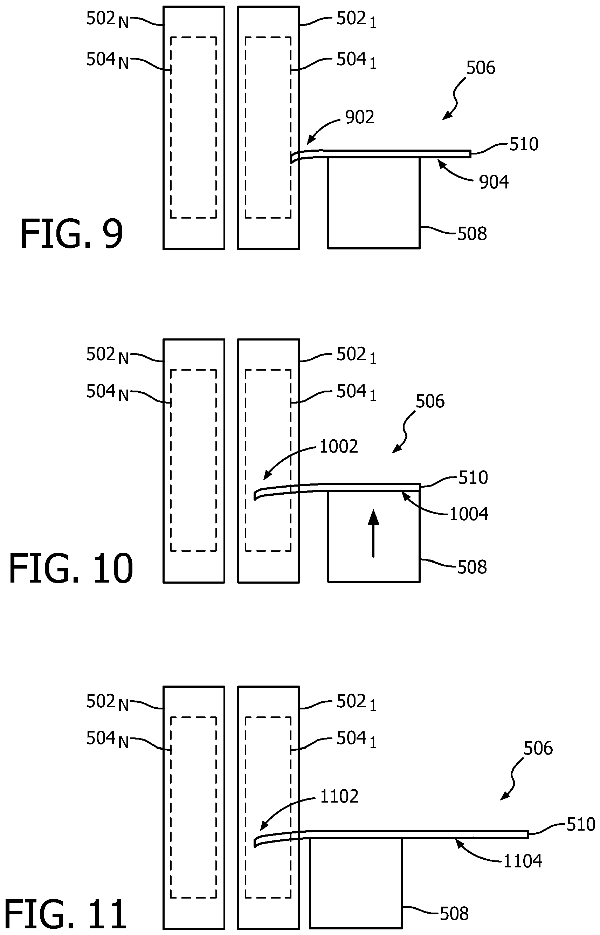

With vertical height adjustment, the base 508 can be adjusted during data acquisition. By way of non-limiting example, the vertical height can be adjusted to keep the deflection delta below 1.2 millimeters (mm) for every 80 mm of travel in connection with a single one of the scanners 502, such as below 1.0 mm, 0.4 mm, or 0.1 mm, including 0.0 mm. When scanning with at least two of the scanners 502, the vertical height can be adjusted to keep the deflection delta below 2.4 mm for every 650 mm travel in the scan direction, such as below 1.5 mm, 1.0 mm, or 0.1 mm, including 0.0 mm. FIGURES 9 and 10, 11 and 12, and 13 and 14 illustrate non-limiting embodiments.

FIGURES 9 and 10 illustrate an embodiment in which N=2, and the scanner 502₁ is being used to scan. In FIGURE 9, the tabletop 510 is located at a first position 902 and a first height 904 in the examination region 504₁. In FIGURE 10, the tabletop 510 is located at a second position 1002 and a second height 1004 in the examination region 504_{N}, which compensates for the deflection delta between the first position 902 and the second position 1002.

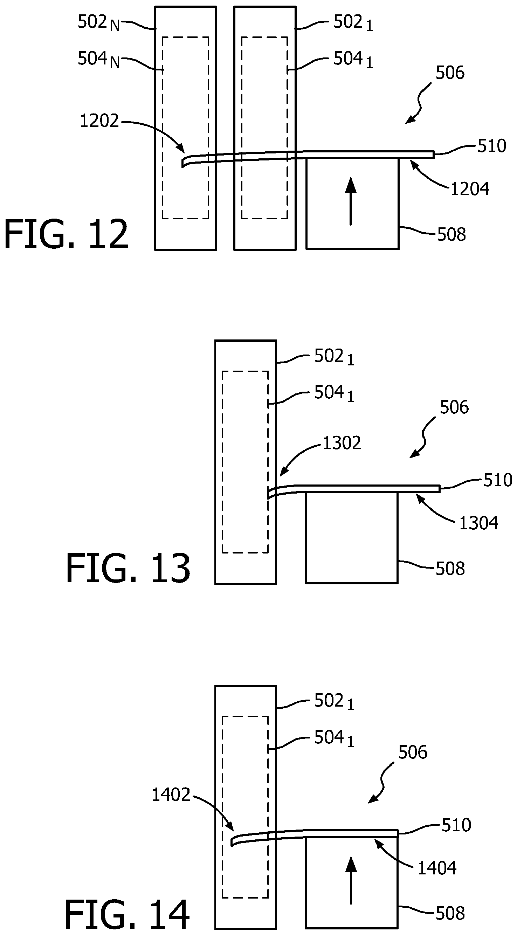

In FIGURE 11, the tabletop 510 is located at a position 1102 and a first height 1104 in the examination region 504₁. In FIGURE 12, the tabletop 510 is located at a corresponding position 1202 and a second height 1204, which compensates for the deflection delta, between the examination regions 504₁ and 504_{N}.

FIGURES 13 and 14 illustrate an embodiment in which N=1. In FIGURE 13, the tabletop 510 is located at a position 1302 and a first height 1304 in the examination region 504₁, and in FIGURE 14, the tabletop 510 is located at a corresponding position 1402 and a second height 1404, which compensates for the deflection delta, between the examination regions 504₁ and 504_{N}.

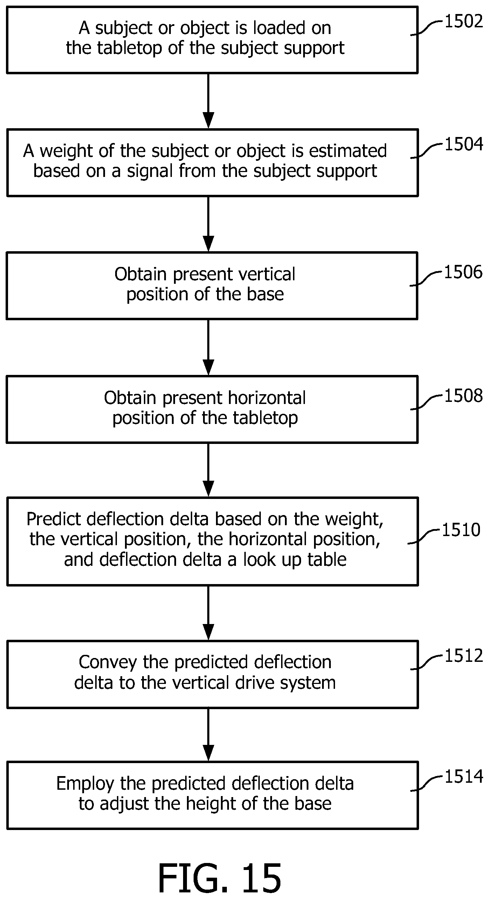

FIGURES 15, 16, and 17 illustrate methods for correcting for a deflection delta.

It is to be appreciated that the ordering of the acts herein is not limiting. As such, other orderings are contemplated herein. In addition, one or more acts may be omitted and/or one or more additional acts may be included.

FIGURE 15 illustrates an example method for correcting for a deflection delta by adjusting a height of a subject support base and hence tabletop.

At 1502, a subject or object is loaded on the tabletop 510 of the subject support 506.

At 1504, a weight of the subject or object on the tabletop 510 is estimated based on a signal from vertical drive system 512 as described herein.

At 1506, a present vertical position of the base 508 is obtained.

At 1508, a present horizontal position of the tabletop 510 is obtained.

At 1510, a deflection delta is predicted based on the weight, the vertical position, the horizontal position, and the LUT 524, as described herein and/or otherwise.

At 1512, the predicted deflection delta is conveyed to the vertical drive system 512.

At 1514, the vertical drive system 512 employs the predicted deflection delta to adjust the height of the base 508.

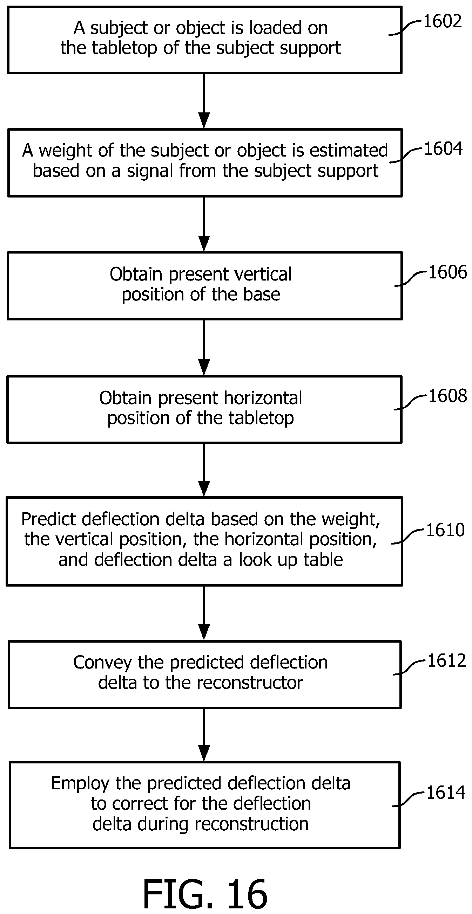

FIGURE 16 illustrates an example method for correcting for a deflection delta in reconstruction.

At 1602, a subject or object is loaded on the tabletop 510 of the subject support 506.

At 1604, a weight of the subject or object on the tabletop 510 is estimated based on a signal from the vertical drive system 512 as described herein.

At 1606, a present vertical position of the base 508 is obtained.

At 1608, a present horizontal position of the tabletop 510 is obtained.

At 1610, a deflection delta is predicted based on the weight, the vertical position, the horizontal position, and the LUT 524, as described herein and/or otherwise.

At 1612, the predicted deflection delta is conveyed to the reconstructor 518 along with the raw data.

At 1614, the reconstructor 518 employs the predicted deflection delta to correct for the deflection delta during image reconstruction.

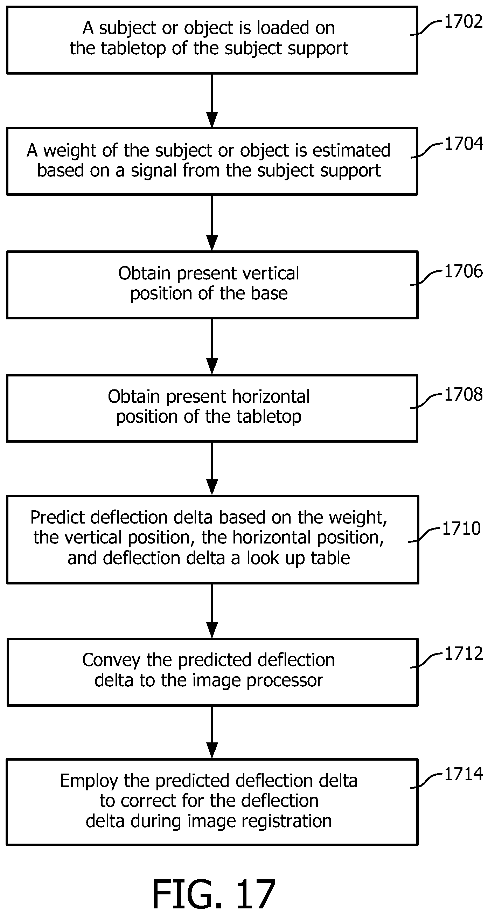

FIGURE 17 illustrates an example method for correcting for a deflection delta for image registration.

At 1702, a subject or object is loaded on the tabletop 510 of the subject support 506.

At 1704, a weight of the subject or object on the tabletop 510 is estimated based on a signal from the vertical drive system 512 as described herein.

At 1706, a present vertical position of the base 508 is obtained.

At 1708, a present horizontal position of the tabletop 510 is obtained.

At 1710, a deflection delta is predicted based on the weight, the vertical position, the horizontal position, and the LUT 524, as described herein and/or otherwise.

At 1712, the predicted deflection delta is conveyed to the image processor 520 along with the reconstructed images.

At 1714, the image processor 520 employs the predicted deflection delta to correct for the deflection during image registration.

The above methods may be implemented by way of computer readable instructions, encoded or embedded on computer readable storage medium, which, when executed by a computer processor(s), cause the processor(s) to carry out the described acts. Additionally or alternatively, at least one of the computer readable instructions is carried by a signal, carrier wave or other transitory medium.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims.

## Claims

1. An imaging system (500), comprising:
a first scanner (502) with a first examination region (504);
a subject support (506), including:
a tabletop (620) configured to position a subject or object at at least two different positions in the examination region, wherein a deflection of the tabletop is different at the at least two different positions; and
a base (508) configured to position the tabletop vertically in the examination region; and
a vertical drive system (512);
a subject / object weight estimator (526) configured to estimate a weight of a subject or object on the tabletop;
a deflection delta predictor (522) configured to predict a deflection delta of the tabletop between the at least two different positions based at least on the weight,
wherein the predicted deflection delta is used to correct for the deflection delta, **characterized in that** the subject / object weight estimator (526) is configured to estimate a weight of a subject or object on the tabletop based at least on an electrical characteristic of the vertical drive system.

2. The imaging system of claim 1, wherein the vertical drive system is configured to adjust a height of the tabletop based on the predicted deflection delta, thereby maintaining an approximate same vertical position of a region of the tabletop at the at least two different positions.

3. The imaging system of claim 1, further comprising:
a reconstructor (518), wherein the reconstructor is configured to correct for the deflection delta during reconstruction based on the predicted deflection delta.

4. The imaging system of any of claims 1 to 3, wherein the deflection delta predictor is configured to predict the deflection delta based on the weight, a present horizontal position of the tabletop, a present vertical position of the base, and a predetermined deflection data look up table, which includes a deflection delta value for different combinations of weight, horizontal position of the tabletop, vertical position of the base.

5. The imaging system of claim 4, further comprising:
at least two transducers (526) that are configured to sense a force exerted by the subject or object on the tabletop and produce an electrical signal indicative thereof,
wherein the subject / object weight estimator is configured to estimate the weight additionally based on the signal.

6. The imaging system of claim 4, further comprising:
a horizontal drive system (514), wherein the horizontal drive system is configured to position the tabletop in the examination region; and
at least one transducers (526) that is configured to sense a force exerted by the subject or object on the tabletop on the horizontal vertical drive system and produce an electrical signal indicative thereof,
wherein the subject / object weight estimator is configured to estimate the weight additionally based on the signal.

7. The imaging system of any of claims 1 to 6, further comprising:
at least a second scanner with a second examination region,
wherein the tabletop is configured to position the subject or object at a first positions in the examination region and a corresponding position in the second examination region, wherein a deflection of the tabletop is different at the first position and the corresponding position, and the predicted deflection delta corresponds to the first position and the corresponding position.

8. The imaging system of claim 7, further comprising:
an image processor (520), wherein the image processor is configured to correct for the deflection delta during image registration of images from the different scanners based on the predicted deflection delta.

9. The imaging system of any of claims 1 to 8, wherein the electrical characteristic is a motor current of a motor of the vertical drive system.

10. The imaging system of claim 9, wherein the subject / object weight estimator is configured to extract the weight from a constant speed vertical motion current.

11. The imaging system of claim 9, further comprising:
a horizontal drive system (514), wherein the subject / object weight estimator is configured to additionally extract the weight from a horizontal motor current during an acceleration and a deceleration phase.

12. A method, comprising:
estimating a weight of a subject or object on a tabletop of a subject support of an imaging system based at least on an electrical characteristic of a vertical drive system of the subject support;
obtaining a vertical position of a base of the subject support;
obtaining a horizontal position of the tabletop in the examination region;
predicting a deflection delta of the tabletop based on the weight, the vertical position, and the horizontal position; and
correcting for a deflection delta for the tabletop based on the predicted deflection delta.

13. The method of claim 12, where the electrical characteristic is a motor current of a motor of the vertical drive system.

14. The method of claim 13, further comprising:
engaging a vertical holding brake;
applying a static holding current;
releasing the vertical holding brake;
servoing the motor to hold the vertical position;
sampling the motor current;
averging the sampled motor current; and
comparing the average with a calibrated value to extract the weight.

15. The method of claim 12, further comprising: additionally extracting the weight from a horizontal motor current during an acceleration and a deceleration phase.

## Patentansprüche

1. Ein Abbildungssystem (500), das Folgendes umfasst:
einen ersten Scanner (502) mit einem ersten Untersuchungsbereich (504);
eine Subjekt-Halterung (506), die Folgendes umfasst:
eine Tischplatte (620), die so eingerichtet ist, dass sie ein Subjekt oder Objekt an mindestens zwei verschiedenen Positionen im Untersuchungsbereich positioniert, wobei eine Auslenkung der Tischplatte an den mindestens zwei verschiedenen Positionen unterschiedlich ist; und
einen Sockel (508), der so konfiguriert ist, dass die Tischplatte vertikal im Untersuchungsbereich positioniert wird; und
ein vertikales Antriebssystem (512);
einen Subjekt-/Objekt-Gewichtsschätzer (526), der so konfiguriert ist, dass er das Gewicht eines Subjekts oder Objekts auf der Tischplatte schätzen kann einen Durchbiegungs-Delta-Prädiktor (522), der so konfiguriert ist, dass er ein Durchbiegungs-Delta der Tischplatte zwischen den mindestens zwei verschiedenen Positionen auf der Grundlage mindestens des Gewichts vorhersagt,
wobei das vorhergesagte Durchbiegungs-Delta verwendet wird, um das Durchbiegungs-Delta zu korrigieren, was **dadurch gekennzeichnet ist, dass** der Subjekt-/Objekt-Gewichtsschätzer (526) dazu konfiguriert ist, ein Gewicht eines Subjekts oder Objekts auf der Tischplatte basierend auf mindestens einer elektrischen Charakteristik des vertikalen Antriebssystems zu schätzen.

2. Das Abbildungssystem nach Anspruch 1, wobei das vertikale Antriebssystem dazu konfiguriert ist, eine Höhe der Tischplatte basierend auf dem vorhergesagten Durchbiegungs-Delta einzustellen, wodurch eine ungefähr gleiche vertikale Position eines Bereichs der Tischplatte an den mindestens zwei verschiedenen Positionen aufrechterhalten wird.

3. Das Abbildungssystem nach Anspruch 1, das ferner Folgendes umfasst:
einen Rekonstruktor (518), wobei der Rekonstruktor dazu konfiguriert ist, das Durchbiegungs-Delta während der Rekonstruktion basierend auf dem vorhergesagten Durchbiegungs-Delta zu korrigieren.

4. Das Abbildungssystem nach einem der Ansprüche 1 bis 3, wobei der Durchbiegungs-Delta-Prädiktor dazu konfiguriert ist, das Durchbiegungs-Delta vorherzusagen auf Grundlage des Gewichts, einer gegenwärtigen horizontalen Position der Tischplatte, einer gegenwärtigen vertikalen Position des Sockels und einer vorbestimmten Durchbiegungsdaten-Nachschlagtabelle, die einen Durchbiegungs-Deltawert für verschiedene Kombinationen von Gewicht, horizontaler Position der Tischplatte, vertikaler Position des Sockels enthält.

5. Das Abbildungssystem nach Anspruch 4, das ferner Folgendes umfasst:
mindestens zwei Wandler (526), die dazu konfiguriert sind, eine von dem Subjekt oder Objekt auf die Tischplatte ausgeübte Kraft zu erfassen und ein elektrisches Signal zu erzeugen, das dafür kennzeichnend ist;
wobei der Subjekt-/ Objekt-Gewichtsschätzer dazu konfiguriert ist, das Gewicht zusätzlich basierend auf Grundlage des Signals zu schätzen.

6. Das Abbildungssystem nach Anspruch 4, das ferner Folgendes umfasst:
ein horizontales Antriebssystem (514), wobei das horizontale Antriebssystem dazu konfiguriert ist, die Tischplatte in dem Untersuchungsbereich zu positionieren; und
zumindest einen Wandler (526), der dazu konfiguriert ist, eine von dem Subjekt oder Objekt auf die Tischplatte auf das horizontale vertikale Antriebssystem ausgeübte Kraft zu erfassen und ein elektrisches Signal zu erzeugen, das dafür kennzeichnend ist;
wobei der Subjekt-/ Objekt-Gewichtsschätzer dazu konfiguriert ist, das Gewicht zusätzlich basierend auf Grundlage des Signals zu schätzen.

7. Das Abbildungssystem nach einem der Ansprüche 1 bis 6, das ferner mindestens einen zweiten Scanner mit einem zweiten Untersuchungsbereich umfasst,
wobei die Tischplatte dazu ausgelegt ist, das Subjekt oder Objekt an einer ersten Position in dem Untersuchungsbereich und einer entsprechenden Position in dem zweiten Untersuchungsbereich zu positionieren, wobei eine Durchbiegung der Tischplatte an der ersten Position und der entsprechenden Position unterschiedlich ist und das vorhergesagte Durchbiegungs-Delta der ersten Position und der entsprechenden Position entspricht.

8. Das Abbildungssystem nach Anspruch 7, das ferner Folgendes umfasst:
einen Bildprozessor (520), wobei der Bildprozessor dazu konfiguriert ist, das Durchbiegungs-Delta während der Bildaufnahme von Bildern von den verschiedenen Scannern basierend auf dem vorhergesagten Durchbiegungs-Delta zu korrigieren.

9. Das Abbildungssystem nach einem der Ansprüche 1 bis 8, wobei die elektrische Charakteristik ein Motorstrom eines Motors des vertikalen Antriebssystems ist.

10. Das Abbildungssystem nach Anspruch 9, wobei der Subjekt-/Objekt- Gewichtsschätzer dazu ausgelegt ist, das Gewicht aus einer vertikalen Bewegung mit konstanter Geschwindigkeit zu ermitteln.

11. Das Abbildungssystem nach Anspruch 9, das ferner Folgendes umfasst:
ein horizontales Antriebssystem (514), bei dem der Subjekt-/ Objekt-Gewichtsschätzer zusätzlich dazu konfiguriert ist, das Gewicht aus einem horizontalen Motorstrom während einer Beschleunigungs- und einer Verzögerungsphase zu extrahieren.

12. Ein Verfahren, das Folgendes umfasst:
Schätzung eines Gewichts eines Subjekts oder Objekts auf einer Tischplatte einer Subjekt-Halterung eines Abbildungssystems basierend auf mindestens einer elektrischen Charakteristik eines vertikalen Antriebssystems der Subjekt-Halterung;
Erlangung einer vertikalen Position eines Sockels der Subjekt-Halterung;
Erlangung einer horizontalen Position der Tischplatte im Untersuchungsbereich;
Vorhersage eines Durchbiegungs-Deltas der Tischplatte auf Grundlage des Gewichts, der vertikalen Position und der horizontalen Position; und
Korrektur eines Durchbiegungs-Deltas für die Tischplatte auf Grundlage des vorhergesagten Durchbiegungs-Deltas.

13. Das Verfahren nach Anspruch 12, wobei die elektrische Charakteristik ein Motorstrom eines Motors des vertikalen Antriebssystems ist.

14. Das Verfahren nach Anspruch 13, das ferner Folgendes umfasst:
Betätigung einer vertikalen Haltebremse;
Anlegen eines statischen Haltestroms;
Lösen der vertikalen Haltebremse;
Servo-Steuerung des Motors, um die vertikale Position zu halten;
Abtastung des Motorstroms;
Mittelung des abgetasteten Motorstroms; und
Vergleich des Durchschnitts mit einem kalibrierten Wert, um das Gewicht zu ermitteln.

15. Das Verfahren nach Anspruch 12, das ferner Folgendes umfasst: zusätzliche Ermittlung des Gewichts aus einem horizontalen Motorstrom während einer Beschleunigungs- und einer Verzögerungsphase.

## Revendications

1. Système d'imagerie (500), comprenant:
un premier balayeur (502) comportant une première zone d'examen (504);
un support de sujet (506), comprenant:
un plateau de table (620) conçu pour positionner un sujet ou un objet à au moins deux positions différentes dans la zone d'examen, dans lequel une déviation du plateau de table est différente à l'au moins deux positions différentes; et
une base (508) conçue pour positionner le plateau de table verticalement dans la zone d'examen; et
un système d'entraînement vertical (512);
un estimateur de poids (526) du sujet/objet conçu pour estimer le poids d'un sujet ou d'un objet sur le tableau de table;
un prédicteur (522) de delta de déviation conçu pour prédire un delta de déviation du plateau de table entre l'au moins deux positions différentes en fonction d'au moins le poids,
dans lequel le delta de déviation prédit est utilisé pour corriger le delta de déviation, **caractérisé en ce que** l'estimateur de poids (526) du sujet/objet est conçu pour estimer le poids d'un sujet ou d'un objet sur le plateau de table en fonction d'au moins une caractéristique électrique du système d'entraînement vertical.

2. Système d'imagerie selon la revendication 1, dans lequel le système d'entraînement vertical est conçu pour ajuster une hauteur du plateau de table en fonction du delta de déviation prédit, maintenant ainsi approximativement la même position verticale d'une zone du plateau de table dans l'au moins deux positions différentes.

3. Système d'imagerie selon la revendication 1, comprenant en outre:
un reconstructeur (518), dans lequel ledit reconstructeur est conçu pour corriger le delta de déviation lors de la reconstruction en fonction du delta de déviation prédit.

4. Système d'imagerie selon l'une quelconque des revendications 1 à 3, dans lequel le prédicteur de delta de déviation est conçu pour prédire le delta de déviation en fonction du poids, d'une position horizontale actuelle du plateau de table, d'une position verticale actuelle de la base et d'une table de recherche de données de déviation prédéterminée, laquelle comprend une valeur delta de déviation pour différentes combinaisons de poids, la position horizontale du plateau de table, la position verticale de la base.

5. Système d'imagerie selon la revendication 4, comprenant en outre:
au moins deux transducteurs (526), lesquels sont conçus pour détecter une force exercée par le sujet ou par l'objet sur le plateau de table et pour produire un signal électrique indicatif de ladite force, dans lequel l'estimateur de poids du sujet/objet est conçu pour estimer le poids en plus en fonction du signal.

6. Système d'imagerie selon la revendication 4, comprenant en outre:
un système d'entraînement horizontal (514), dans lequel le système d'entraînement horizontal est conçu pour positionner le plateau de table dans la zone d'examen; et
au moins un transducteur (526), lequel est conçu pour détecter une force exercée par le sujet ou par l'objet sur le plateau de table sur le système d'entraînement vertical/horizontal et pour produire un signal électrique indicatif de ladite force,
dans lequel l'estimateur de poids du sujet/objet est conçu pour estimer le poids en plus en fonction du signal.

7. Système d'imagerie selon l'une quelconque des revendications 1 à 6, comprenant en outre:
au moins un second balayeur comportant une seconde zone d'examen,
dans lequel le plateau de table est conçu pour positionner le sujet ou l'objet à une première position dans la zone d'examen et à une position correspondante dans la seconde zone d'examen, dans lequel une déviation du plateau de table est différente à la première position et à la position correspondante, et la déviation delta prédite correspond à la première position et à la position correspondante.

8. Système d'imagerie selon la revendication 7, comprenant en outre:
un processeur d'images (520), dans lequel le processeur d'images est conçu pour corriger le delta de déviation lors de l'enregistrement d'images des images provenant de différents balayeurs en fonction du delta de déviation prédit.

9. Système d'imagerie selon l'une quelconque des revendications 1 à 8, dans lequel la caractéristique électrique est un courant de moteur d'un moteur du système d'entraînement vertical.

10. Système d'imagerie selon la revendication 9, dans lequel l'estimateur de poids sujet/objet est conçu pour extraire le poids d'un courant de mouvement vertical à vitesse constante.

11. Système d'imagerie selon la revendication 9, comprenant en outre:
un système d'entraînement horizontal (514), dans lequel l'estimateur de poids du sujet/objet est conçu pour extraire en plus le poids d'un courant de moteur horizontal lors d'une étape d'accélération et de décélération.

12. Procédé, consistant:
à estimer un poids d'un sujet ou d'un objet sur un plateau de table d'un support de sujet d'un système d'imagerie en fonction d'au moins une caractéristique électrique d'un système d'entraînement vertical du support de sujet;
à obtenir une position verticale d'une base du support de sujet;
à obtenir une position horizontale du plateau de table dans la zone d'examen;
à prédire un delta de déviation du plateau de table en fonction du poids, de la position verticale et de la position horizontale; et
à corriger un delta de déviation pour le plateau de table en fonction du delta de déviation prédit.

13. Procédé selon la revendication 12, dans lequel la caractéristique électrique est un courant de moteur d'un moteur du système d'entraînement vertical.

14. Procédé selon la revendication 13, consistant en outre:
à engager un frein de maintien vertical;
à appliquer un courant de maintien statique;
à desserrer le frein de maintien vertical;
à asservir le moteur pour maintenir la position verticale;
à échantillonner le courant de moteur;
à moyenner le courant de moteur échantillonné; et
à comparer la moyenne avec une valeur échantillonnée pour extraire le poids.

15. Procédé selon la revendication 12, consistant en outre:
à extraire en plus le poids d'un courant de moteur horizontal lors d'une étape d'accélération et de décélération.
